# EUROPÄISCHE PATENTSCHRIFT

(11) **EP 0 441 183 B1**
(45) Veröffentlichungstag und Bekanntmachung des Hinweises auf die Patenterteilung: **03.11.1993**
(21) Anmeldenummer: 91100864.7
(22) Anmeldetag: 24.01.1991
(51) Int. Cl.: C07C 53/10, C07C 53/126, C07C 53/128, C07C 51/41

(54) **Verfahren zur Herstellung von wasserfreien Zinn(IV)-carboxylaten**
Process for preparing anhydrous tin(IV)-carboxylates
Procédé de préparation de carboxylates d'étain(IV) anhydres

(30) Priorität: 06.02.1990 DE 4003488
(43) Veröffentlichungstag der Anmeldung: 14.08.1991
(73) Patentinhaber: Th. Goldschmidt AG, 45127 Essen (DE)
(72) Erfinder: Ruf, Erich, Dr., W-4300 Essen (DE)

(56) Entgegenhaltungen:
- EP-A- 0 166 048
- GB-A- 600 722

## Beschreibung

Die Erfindung betrifft ein Verfahren zur Herstellung von wasserfreien Zinn(IV)-carboxylaten.

Aus der Literatur (Gmelin, 8. Auflage, 1975, Nr. 46, Teil C 2, Seiten 220, 221) ist die Umsetzung von metallischem Zinn und Eisessig während 80- bis 90stündigem Erhitzen im Rückfluß unter Inertgas bekannt, bei der nur Zinn(II)-acetat erhalten wird.

Zur Herstellung von Zinn(IV)-acetat benötigt man teure Zwischenverbindungen, wie z. B. Thalliumacetat. Dieses wird in Essigsäureanhydrid mit Zinn(IV)-jodid oder -bromid zu Zinn(IV)-acetat umgesetzt. Ein solches Verfahren ist umständlich und wirtschaftlich sehr aufwendig.

Der Erfindung liegt die Aufgabe zugrunde, ein Verfahren zu finden, das die Herstellung von wasserfreien Zinn(IV)-carboxylaten in einfacher und wirtschaftlicher Weise ermöglicht.

Diese Aufgabe wird durch das erfindungsgemäße Verfahren gelöst. Das Verfahren ist dadurch gekennzeichnet, daß man zunächst entweder metallisches Zinn oder Sn(II)-acetat mit Essigsäureanhydrid im Überschuß unter Rühren bei Temperaturen von 50 bis 150°C unter Durch leiten von Sauerstoff oder Zusatz von sauerstoffabgebenden Mitteln behandelt, das entstandene Zinn(IV)-acetat abtrennt und dieses dann in an sich bekannter Weise mit organischen Säureanhydriden oder Carbonsäuren mit mehr als vier Kohlenstoffatomen bei Temperaturen von 80 bis 150°C umsetzt und das freigesetzte Essigsäureanhydrid bzw. die freigesetzte Essigsäure unter Vakuum abdestilliert.

Das erfindungsgemäße Verfahren läuft somit in zwei Stufen ab: In der ersten Stufe wird Zinn(IV)-acetat hergestellt, das in der zweiten Verfahrensstufe zur Bildung der gewünschten Carboxylate mit den entsprechenden Säureanhydriden oder Carbonsäuren umgeestert wird.

### 1. Verfahrensstufe

Das metallische Zinn wird zur Erhöhung seiner Reaktivität vorzugsweise in feingepulverter Form eingesetzt. Die Umsetzung des metallischen Zinns oder des Sn(II)-acetates erfolgt bei 50 bis 150°C, bevorzugt ist ein Bereich von 80 bis 130°C. Dabei hat es sich als günstig herausgestellt, die Umsetzung in Gegenwart von Essigsäure als Lösungsmittel durchzuführen.

Die Oxidation des Zinns in die vierwertige Stufe erfolgt mit Sauerstoff oder sauerstoffabgebenden Mitteln.

Als sauerstoffabgebendes Mittel verwendet man zweckmäßigerweise eine wäßrige Wasserstoffperoxidlösung. Um dabei das gewünschte wasserfreie Zinn(IV)-acetat zu erhalten, ist es notwendig, das über die Wasserstoffperoxidlösung eingebrachte Wasser durch Zugabe von entsprechenden Mengen an Essigsäureanhydrid in Essigsäure zu überführen. Nach einer Reaktionszeit von 5 bis 8 Stunden wird das Reaktionsgemisch abgekühlt, das ausgefallene Zinn(IV)-acetat abfiltriert und getrocknet. Das weiße, wasserfreie Zinn(IV)-acetat fällt dabei in einer Ausbeute von 70 bis 80 % an. Durch Einengen und/oder Abkühlen der Mutterlauge kann weiteres Zinn(IV)-acetat gewonnen werden, so daß hierdurch die Gesamtausbeute auf ca. 97 % erhöht wird.

### 2. Verfahrensstufe

In der zweiten Verfahrensstufe erfolgt die Umsetzung des in der ersten Stufe erhaltenen wasserfreien Zinn(IV)-acetats mit organischen Säureanhydriden oder Carbonsäuren mit mehr als vier Kohlenstoffatomen bei Temperaturen von 80 bis 150°C bei gleichzeitigem Abdestillieren des dabei freigesetzten Essigsäureanhydrids bzw. der freigesetzten Essigsäure unter vermindertem Druck. Dabei werden auf einfache Weise die entsprechenden Zinn(IV)carboxylate in wasserfreier Form erhalten.

In einer bevorzugten Ausführung läßt man die Umsetzung von Zinn(IV)-acetat mit dem organischen Säureanhydrid bzw. mit der organischen Säure bei Temperaturen von 100 bis 110°C unter leichtem Vakuum ablaufen. In nahezu quantitativer Ausbeute fällt dabei das Zinn(IV)-carboxylat des jeweils eingesetzten organischen Säureanhydrids an.

Als organische Säureanhydride mit mehr als vier Kohlenstoffatomen können unter anderem Propionsäureanhydrid, Buttersäureanhydrid und Valeriansäureanhydrid eingesetzt werden. Besonders bevorzugt werden die Anhydride der höhermolekularen Fettsäuren, wie z. B. der Laurinsäure, der Palmitinsäure oder der Stearinsäure, verwendet. Als Carbonsäure mit mehr als vier Kohlenstoffatomen können Valeriansäure, Caprylsäure oder Undecansäure ausgewählt werden. Auch hier werden vorzugsweise die höhermolekularen Säuren, wie Myristinsäure, Palmitinsäure oder Stearinsäure, verwendet. Beispiele für ungesättigte Fettsäuren sind die Ölsäure und die Linolsäure. Ganz allgemein sind Fettsäuren mit bis zu 22 Kohlenstoffatomen oder deren Anhydride bevorzugt.

Das in der ersten Stufe des erfindungsgemäßen Verfahrens hergestellte wasserfreie Zinn(IV)-acetat kann als Zinnbasisverbindung beim Aufbringen von elektrisch leitenden und infrarotreflektierenden Schichten auf Glas- oder Keramikoberflächen eingesetzt werden. Darüber hinaus kann Zinn(IV)-acetat als solches bzw. in Formulierungen für die chemische Konservierung von Glasoberflächen verwendet werden. Die höhermolekularen Zinn(IV)-carboxylate eignen sich als Gleit- und Schmiermittel sowie als Preßhilfe bei Sinterwerkstoffen, insbesondere bei solchen auf Bronzebasis. Ein weiteres Einsatzgebiet für Zinn(IV)-acetat ist die Herstellung von zinnhaltigen Metalloxanverbindungen.

In den folgenden Beispielen wird das erfindungsgemäße Verfahren näher erläutert. Dabei wird in den Beispielen 1 bis 4 die Herstellung des wasserfreien Zinn(IV)-acetats (Stufe 1 des erfindungsgemäßen Verfahrens) und in den Beispielen 5 bis 11 die Herstellung der Zinn(IV)-carboxylate (Stufe 2 des erfindungsgemäßen Verfahrens) beschrieben.

### Beispiel 1

In einem 500-ml-Vierhalskolben mit Thermometer, Kühler, Rührer und Gaseinleitrohr werden
- 35,6 g: Zinnpulver
- 67,3 g: Essigsäureanhydrid und
- 225,0 g: Essigsäure

vorgelegt und unter Rühren auf ca. 110°C aufgeheizt. Durch dieses aufgeheizte Stoffgemisch wird Sauerstoff geleitet. Die Reaktionszeit bei stetem Einleiten von Sauerstoff beträgt ca. 6,5 Std. Anschließend wird die Essigsäure mit Hilfe eines Rotationsverdampfers bei ca. 20 mm Hg/ca. 60°C im Vakuum abdestilliert. Das hierbei anfallende Zinn(IV)-acetat wird mit 300 g Essigsäureanhydrid versetzt und die Mischung kurz auf ca. 100°C aufgeheizt. Im Anschluß daran wird Essigsäureanhydrid in einem Rotationsverdampfer im Vakuum bei 10 mm Hg/60°C abdestilliert. Das hierbei erhaltene wasserfreie Zinn(IV)-acetat wird zweimal mit jeweils ca. 75 ml Ethylacetat gewaschen und im Rotationsverdampfer getrocknet.
Ausbeute: 103,7 g wasserfreies Zinn(IV)-acetat

### Beispiel 2

In einem 500-ml-Vierhalskolben mit Thermometer, Kühler, Rührer und Gaseinleitrohr werden
- 23,7 g: Zinnpulver und
- 255,0 g: Essigsäureanhydrid

vorgelegt und unter Rühren bis zum Rückfluß erhitzt. Anschließend wird durch das Stoffgemisch bei 120°C Sauerstoff geleitet. Nach ca. 1 Std. werden zu dieser Stoffmischung 300 g Essigsäureanhydrid gegeben und weiterhin Sauerstoff bei ca. 120°C eingeleitet. Die Reaktionszeit beträgt ca. 4 Std. Die hierbei erhaltene Lösung wird auf 0°C abgekühlt, über eine Glasfritte filtriert; der hierbei anfallende Filterrückstand wird mit ca. 75 ml Ethylacetat gewaschen und im Rotationsverdampfer getrocknet.
Ausbeute: 52 g wasserfreies Zinn(IV)-acetat

### Beispiel 3

In einem 2-1-Vierhalskolben mit Thermometer, Rührer, Kühler und Tropftrichter werden
- 1020 g: Essigsäureanhydrid
- 95 g: Zinnpulver

vorgelegt und auf ca. 140°C aufgeheizt. Nach 1 Std. wird diese Produktmischung auf eine Temperatur von ca. 110 bis 120°C gebracht und mit 88 g 70 %iger Wasserstoffperoxidlösung tropfenweise versetzt. Nach ca. 3 Std. Eintropfzeit wird das Reaktionsgemisch mit 300 g Essigsäureanhydrid versetzt und 1 Std. bei 120°C und dann bis zum Erreichen der Raumtemperatur gerührt.

Das hierbei anfallende Zinn(IV)-acetat wird abfiltriert. Die Filtratlösung wird auf ca. 4°C abgekühlt, wodurch weiteres Zinn(IV)-acetat ausgeschieden wird. Dieses ausgefallene Zinn(IV)-acetat wird ebenfalls abfiltriert und zusammen mit der ersten Filtratmenge dreimal mit ca. 75 ml Butylacetat gewaschen. Das gewaschene Zinn(IV)-acetat wird anschließend bei Raumtemperatur im Vakuum bei 2 mm Hg in einem Rotationsverdampfer getrocknet.
Ausbeute: 227,6 g wasserfreies Zinn(IV)-acetat

### Beispiel 4

In einem 1-l-Vierhalskolben mit Thermometer, Rührer, Kühler und Gaseinleitrohr werden
- 47,3 g: Zinn(II)-acetat
- 44,9 g: Essigsäureanhydrid und
- 150,1 g: Essigsäure

vorgelegt und unter Rühren auf 115°C aufgeheizt. Durch dieses aufgeheizte Stoffgemisch wird Sauerstoff geleitet. Die Reaktionszeit beträgt ca. 2,5 Std. Anschließend wird die Reaktionsmischung am Rotationsverdampfer bei 60 bis 80°C im Vakuum (< 10 mbar) von den flüssigen Bestandteilen befreit. Der Gehalt an Sn(II)-Verbindungen beträgt ca. 0,1 %.
Ausbeute: 65,4 g wasserfreies Zinn(IV)-acetat

### Beispiel 5

In einem 250-ml-Vierhalskolben mit Thermometer, Kühler und Rührer werden
- 35,5 g: Zinn(IV)-acetat und
- 113,8 g: Stearinsäure

vorgelegt und unter leichtem Vakuum auf 100°C aufgeheizt. Bei ca. 70°C erhält man eine klare Schmelze. Die sich bildende Essigsäure wird im Vakuum (20 bis 2 mm Hg/100°C) abdestilliert und in Kühlfallen aufgefangen. In ca. 6,5 Std. ist die Essigsäure quantitativ abdestilliert.
Ausbeute: 124,8 g Zinn(IV)-stearat

### Beispiel 6

In einem 250-ml-Vierhalskolben mit Thermometer, Kühler und Rührer werden
- 35,5 g: Zinn(IV)-acetat und
- 110,2 g: Stearinsäureanhydrid

vorgelegt und unter leichtem Vakuum auf 100°C aufgeheizt. Bei ca. 70°C erhält man eine klare Schmelze. Anschließend wird Essigsäureanhydrid im Vakuum (20 bis 2 mm Hg/100°C) abdestilliert und in Kühlfallen aufgefangen. Nach ca. 6,5 Std. ist praktisch die gesamte Menge an Essigsäureanhydrid abdestilliert.
Ausbeute: 127 g Zinn(IV)-stearat

### Beispiel 7

In einem 250-ml-Vierhalskolben mit Thermometer, Kühler und Rührer werden
- 17,7 g: Zinn(IV)-acetat und
- 90,4 g: Montansäure

(aliphatische Carbonsäure mit ca. 32 C-Atomen) vorgelegt und unter leichtem Vakuum auf 100°C aufgeheizt. Bei ca. 80°C erhält man eine gelbliche Schmelze. Dann wird Essigsäure im Vakuum bei ca. 20 bis 2 mm Hg/100°C abdestilliert und in Kühlfallen aufgefangen (Dauer ca. 6 Std).
Ausbeute: 96,2 g Zinn(IV)-montanat

### Beispiel 8

In einem 250-ml-Vierhalskolben mit Rührer, Destillationsaufsatz und Thermometer werden
- 35,5 g: Zinn(IV)-acetat und
- 46,5 g: Capronsäure

vorgelegt. Die Mischung wird unter Rühren auf 110°C erhitzt. Nach Anlegen eines leichten Vakuums wird die entstehende Essigsäure abdestilliert und in einer gekühlten Vorlage aufgefangen. Die Reaktionszeit beträgt ca. 2,5 Std.
Ausbeute: 57,0 g Zinn(IV)-capronat

### Beispiel 9

In einem 250-ml-Vierhalskolben mit Rührer, Destillationsaufsatz und Thermometer werden
- 35,5 g: Zinn(IV)-acetat und
- 58,5 g: 2-Ethylhexansäure

vorgelegt. Die Mischung wird unter Rührer und leichtem Vakuum auf 130°C erhitzt und die entstehende Essigsäure in eine gekühlte Vorlage destilliert. Die Reaktionszeit beträgt ca. 3 Std.
Ausbeute: 69,7 g Zinn(IV)-2-ethylhexanoat

### Beispiel 10

In einem 500-ml-Vierhalskolben mit Rührer, Destillationsaufsatz und Thermometer werden
- 35,5 g: Zinn(IV)-acetat und
- 80,1 g: Laurinsäure

vorgelegt. Ein leichtes Vakuum wird angelegt und die Mischung bei ca. 60°C aufgeschmolzen und anschließend unter Rühren weiter auf 100°C erhitzt. Die entstehende Essigsäure wird in einer gekühlten Vorlage aufgefangen. Die Reaktionszeit beträgt ca. 2,5 Std.
Ausbeute: 93,1 g Zinn(IV)-laurat

### Beispiel 11

In einem 500-ml-Vierhalskolben mit Rührer, Destillationsaufsatz und Thermometer werden
- 37,3 g: Zinn(IV)-acetat und
- 143,1 g: Behensäure

vorgelegt. Die Mischung wird bei ca. 80°C in einem leichten Vakuum aufgeschmolzen und weiter auf 120°C erhitzt. Die entstehende Essigsäure wird in einer gekühlten Vorlage aufgefangen. Die Reaktionszeit beträgt ca. 3 Std.
Ausbeute: 156,2 g Zinn(IV)-behenat

## Patentansprüche

1. Verfahren zur Herstellung von wasserfreien Zinn(IV)-carboxylaten, dadurch gekennzeichnet, daß man zunächst entweder metallisches Zinn oder Sn(II)-acetat mit Essigsäureanhydrid im Überschuß unter Rühren bei Temperaturen von 50 bis 150°C unter Durch leiten von Sauerstoff oder Zusatz von sauerstoffabgebenden Mitteln behandelt, das entstandene Zinn(IV)-acetat abtrennt und dieses dann in an sich bekannter Weise mit organischen Säureanhydriden oder Carbonsäuren mit mehr als vier Kohlenstoffatomen bei Temperaturen von 80 bis 150°C umsetzt und das freigesetzte Essigsäureanhydrid bzw. die freigesetzte Essigsäure unter Vakuum abdestilliert.

2. Verfahren nach Anspruch 1, dadurch gekennzeichnet, daß man pulverförmiges Zinn verwendet.

3. Verfahren nach Anspruch 1 und 2, dadurch gekennzeichnet, daß man in der ersten Verfahrensstufe in Gegenwart von Essigsäure als Lösungsmittel arbeitet.

4. Verfahren nach Anspruch 1 bis 3, dadurch gekennzeichnet, daß man in der ersten Verfahrensstufe bei Temperaturen von 80 bis 130°C und in der zweiten Verfahrensstufe bei Temperaturen von 100 bis 110°C arbeitet.

5. Verfahren nach Ansprüchen 1 bis 4, dadurch gekennzeichnet, daß man als sauerstoffabgebendes Mittel Wasserstoffperoxid verwendet und das dadurch in das Reaktionsgemisch eingebrachte Wasser mit einer entsprechenden Menge Essigsäureanhydrid in Form von Essigsäure bindet.

## Claims

1. Process for the preparation of anhydrous tin(IV) carboxylates, characterised in that first either metallic tin or Sn(II) acetate is treated with acetic anhydride in excess with stirring at temperatures of from 50 to 150°C while oxygen is passed through or with addition of oxygen-releasing agents, the resultant tin(IV) acetate is separated off and then reacted in a manner known per se with organic acid anhydrides or carboxylic acids having more than four carbon atoms at temperatures of from 80 to 150°C, and the liberated acetic anhydride or the liberated acetic acid is removed by distillation in vacuo.

2. Process according to Claim 1, characterised in that pulverulent tin is used.

3. Process according to Claim 1 or 2, characterised in that the first process step is carried out in the presence of acetic acid as solvent.

4. Process according to Claims 1 to 3, characterised in that the first process step is carried out at temperatures of from 80 to 130°C and the second process step is carried out at temperatures of from 100 to 110°C.

5. Process according to Claims 1 to 4, characterised in that the oxygen-releasing agent used is hydrogen peroxide, and the water thereby introduced into the reaction mixture is bound in the form of acetic acid using a corresponding amount of acetic anhydride.

## Revendications

1. Procédé de préparation de carboxylates d'étain (IV) anhydres, caractérisé en ce qu'on traite d'abord de l'étain métallique ou de l'acétate de Sn(II) avec de l'anhydride acétique en excès, sous agitation, à une température comprise entre 50 et 150°C, avec introduction d'oxygène ou addition de substances cédant de l'oxygène, on sépare l'acétate d'étain(IV) formé et ensuite, on fait réagir celui-ci, de manière connue en soi, avec des anhydrides d'acides organiques ou des acides carboxyliques ayant plus de quatre atomes de carbone, à une température comprise entre 80 et 150°C, et on sépare, par distillation sous vide, l'anhydride acétique libéré ou l'acide acétique libéré.

2. Procédé selon la revendication 1, caractérisé en ce qu'on utilise de l'étain pulvérulent.

3. Procédé selon la revendication 1 ou 2, caractérisé en ce qu'on travaille en présence d'acide acétique, comme solvant, lors de la première étape de procédé.

4. Procédé selon l'une des revendications 1 à 3, caractérisé en ce qu'on travaille à des températures de 80 à 130°C lors de la première étape de procédé, et à des températures de 100 à 110°C lors de la deuxième étape de procédé.

5. Procédé selon l'une des revendications 1 à 4, caractérisé en ce qu'on utilise du peroxyde d'hydrogène comme produit cédant de l'oxygène, et on fixe l'eau ainsi introduite dans le mélange de réaction sous la forme d'acide acétique avec une quantité correspondante d'anhydride acétique.
